# EUROPÄISCHE PATENTSCHRIFT

(11) **EP 4 245 395 B1**
(45) Veröffentlichungstag und Bekanntmachung des Hinweises auf die Patenterteilung: **23.04.2025**
(21) Anmeldenummer: 23161835.6
(22) Anmeldetag: 14.03.2023
(51) Int. Cl.: B01D 19/00, B01F 23/23, C12M 1/34, C02F 9/00

(54) **VORRICHTUNG UND VERFAHREN ZUM KONTINUIERLICHEN GASAUSTAUSCH IN EINEM STROM EINES FLUIDGEMISCHS**
DEVICE AND METHOD FOR CONTINUOUS GAS EXCHANGE IN A FLOW OF A FLUID MIXTURE
DISPOSITIF ET PROCÉDÉ D'ÉCHANGE CONTINU DE GAZ DANS UN FLUX D'UN MÉLANGE DE FLUIDES

(30) Priorität: 17.03.2022 DE 102022106285
(43) Veröffentlichungstag der Anmeldung: 20.09.2023
(73) Patentinhaber: Messer SE & Co. KGaA, 65812 Bad Soden (DE)
(72) Erfinder: Berger, Thomas, 45257 Essen (DE); Herzog, Dr. Friedhelm, 47803 Krefeld (DE); Rohovec, Joachim, 2500 Baden bei Wien (AT)
(74) Vertreter: Münzel, Joachim R.

(56) Entgegenhaltungen:
- WO-A1-03/002227
- WO-A1-2006/032427
- WO-A1-2011/105596
- WO-A1-2022/218636
- WO-A1-2023/011843

## Beschreibung

Die Erfindung betrifft eine Vorrichtung zum kontinuierlichen Gasaustausch in einem Strom eines Fluidgemischs. Die Erfindung betrifft des Weiteren ein entsprechendes Verfahren.

Viele biologische, lebensmitteltechnische oder medizinische Anwendungen verlangen den Austausch von in Flüssigkeiten enthaltenen Gasen. Beispielweise verstoffwechseln in bestimmten mikrobiologischen Verfahren in einer Flüssigkeit suspendierte Mikroorganismen ein in der Flüssigkeit gelöstes Gas und sondern ein anderes Gas als Stoffwechselprodukt in die Flüssigkeit ab. Oft wirken die ausgeschiedenen Gase ab einer gewissen Konzentration inhibierend oder sogar toxisch auf die Organismen, weshalb eine Abtrennung dieser Gase unumgänglich ist, gleichzeitig muss dafür gesorgt werden, dass kontinuierlich zum Verstoffwechseln benötigtes Gas zugeführt wird. Beispiele für solche biologischen Verfahren sind etwa die Aufzucht von Mikro- und Makroalgen, von aeroben Bakterien oder von Inokulationskulturen. Ebenfalls wichtig ist ein Gasaustausch beispielsweise für das Wasser in Aquarien und Fischzuchten. Die Lebewesen dort benötigen Sauerstoff, welches sie in gelöster Form zu sich nehmen, und sondern gleichzeitig CO₂ ab.

Die beiden Verfahrensschritte der Abtrennung eines ersten Gases aus einer Flüssigkeit und das Dispergieren eines zweiten Gases in die gleiche Flüssigkeit werden aktuell meist in getrennten Vorrichtungen durchgeführt, die von dem zu behandelnden flüssigen Medium nacheinander durchlaufen werden. Eine solche Vorgehensweise ist jedoch meist mit einem sehr hohen apparativen Aufwand und entsprechend hohen Kosten sowie einem beträchtlichen Platzbedarf verbinden und geht zudem mit einem hohen verfahrens- und prozessleittechnischem Aufwand einher.

Neben Vorrichtungen wie in der WO03/002227 oder der WO2006/032427 beschrieben, die jeweils nur entgasen oder Gas einbringen können und somit kombiniert werden müssen, kommen Membranverfahren zum Einsatz, die in der Lage sind, einen Austausch von gelösten Gasen in einem flüssigen Medium herbeizuführen. Mittels Membranen arbeitende Einrichtungen zum Gasaustausch in Flüssigkeiten sind beispielsweise aus der EP 1 988 984 B1 und den darin zitierten Druckschriften bekannt.

Der Nachteil des Einsatzes von Membranverfahren beim Gasaustausch ist jedoch, dass die Membranen nach einer gewissen Zeit verstopfen oder abgenutzt werden, weshalb bestimmte flüssige Medien, wie beispielsweise Suspensionen, erst gar nicht behandelt werden können und/oder die Membranen in regelmäßigen Zeitabständen ausgetauscht werden müssen. Zudem benötigen sie bei hohen Gasdurchsätzen eine große Austauschfläche, die mit entsprechendem Platzbedarf einhergeht, und/oder die Membran ist im Betrieb einer hohen Druckdifferenz ausgesetzt, die zu einer hohen Beanspruchung der Porenstruktur und damit zu einer raschen Abnutzung führt.

Der Erfindung liegt somit die Aufgabe zu Grunde, ein Möglichkeit zum kontinuierlichen Gasaustausch in flüssigen Medien zu schaffen, die mit einfachen Mitteln zu realisieren, wartungsarm im Betrieb und für eine Vielzahl unterschiedlicher flüssiger Medien geeignet ist.

Gelöst ist die Aufgabe durch eine Vorrichtung mit den Merkmalen des Patentanspruchs 1 und durch ein Verfahren mit den Merkmalen des Anspruchs 10. Vorteilhafte Ausgestaltungen der Erfindung sind in den Unteransprüchen angegeben.

Eine erfindungsgemäße Vorrichtung umfasst eine Düse zum Zuführen eines Fluidgemischs in ein - beim Einsatz der Vorrichtung mit dem Fluidgemisch gefülltes - Volumen, einen im Volumen angeordneten Separator sowie eine in das Volumen einmündende Gaszuführung für ein gleichzeitig mit der Separation des ersten Gases in das Fluidgemisch zu dispergierendes zweites Gas.

Als "Fluidgemisch" oder "flüssiges Medium" soll hier ein wenigstens eine Flüssigkeit und zumindest ein erstes darin enthaltenes Gas aufweisendes Medium verstanden werden, wobei im Fluidgemisch auch weitere Stoffe, wie beispielsweise Feststoffpartikel oder andere gelöste Gase anwesend sein können. Das erste Gas vor der Behandlung und/oder das zweite Gas nach der Behandlung kann/können im Fluidgemisch homogen oder inhomogen verteilt sein, also insbesondere gelöst oder als Dispersion in Form von in der Flüssigkeit fein verteilten Gasblasen vorliegen.

Die Düse weist einen konischen Ringspalt auf, der zwischen einer konischen Innenfläche eines Düsenmantels und einem Führungskegel angeordnet ist und an seiner Spitze mit einer Düsenöffnung in das Volumen ausmündet. Die Düse ist mit einer tangential, jedoch axial beabstandet von der Düsenöffnung, in den konischen Ringspalt einmündenden Fluidzuführung ausgerüstet. Der tangentiale Eintritt in den Ringspalt zwingt dem Fluidgemisch eine rotierende Bewegung auf, deren Winkelgeschwindigkeit bis zur Düsenöffnung hin drastisch zunimmt. Durch die hohe Rotationsgeschwindigkeit wird das Fluidgemisch an der Düsenöffnung als stark verdrallter Fluidstrahl in das Volumen ausgestoßen. Insbesondere ist die Rotationsgeschwindigkeit aufgrund der Führung des Fluidgemisches durch einen Ringspalt höher als bei einem ansonsten gleich großen hohlkegligen Düsenkörper.

Die Breite des konischen Ringspalts, d.h. der Abstand zwischen der Innenwand des Düsenmantels und der Außenwand des Führungskegels, sollte an keiner Stelle größer sein als der Innendurchmesser der Fluidzuführung an ihrer Einmündung in den Ringspalt. Der Ringspalt kann einen spitzen oder auch einen stumpfen Öffnungswinkel aufweisen. Beispielsweise beträgt der Öffnungswinkel zwischen 30° und 180°, bevorzugt zwischen 45° und 170°, besonders bevorzugt zwischen 60° und 135°; die konischen Begrenzungsflächen des Ringspalts müssen jedoch nicht zwingend gerade Mantellinien aufweisen. Der radiale Abstand zwischen der konischen Innenfläche des Düsenmantels und der Außenfläche des Führungskegels ist zwischen der Einmündung der Fluidzuführung und der Spitze des Führungskegels konstant oder verringert sich stetig in Richtung der Düsenöffnung; eine Aufweitung des Abstandes zwischen den Begrenzungsflächen des Ringspalts zwischen Einmündung der Fluidzuführung und der Düsenöffnung, beispielsweise unter Ausbildung einer Mischkammer, ist erfindungsgemäß nicht vorgesehen. Daher verkleinert sich das dem Fluidgemisch im Ringspalt zur Verfügung stehende Volumen stetig bis zu Düsenöffnung, wodurch sich die axiale Geschwindigkeit, ebenso wie die Rotationsgeschwindigkeit, kontinuierlich erhöht.

In dem sich stromab zur Düsenöffnung im Volumen ausbildenden, stark verdrallten Fluidstrahl besteht in radialer Richtung ein starker Druckgradient, wobei der Druck in einem achsennahen Bereich sehr viel niedriger ist als am Rand des Fluidstrahls. Dadurch konzentrieren sich im Fluidgemisch in Form kleiner Gasbläschen enthaltene Gase im Zentrum des Fluidstrahls, während das Fluidgemisch in dessen Außenbereich von diesen Gasen zumindest weitgehend befreit ist. Handelt es sich beim Fluidgemisch um eine Flüssigkeit mit wenigstens einem darin gelösten Gas, kann der Druck im Innern des Fluidstrahls so niedrig werden, dass es zum Ausgasen wenigstens eines Gases kommt, das sich in der Folge im Zentrum des Fluidstrahls anreichert.

Der Separator taucht mit seinem Trennabschnitt in den sich im Volumen stromab zur Düsenöffnung ausbildenden Fluidstrahl ein. Der Trennabschnitt ist so aufgebaut, dass er einen im Fluidstrahl radial innen liegenden Teil des Fluidgemisches vom übrigen Fluidgemisch trennt und über eine vom Trennabschnitt abgehende Ausleitung abführt. Da sich, wie erwähnt, im achsennahen Bereich des stark verdrallten Fluidstrahls gasförmige Bestandteile des Fluidgemisches konzentrieren, besteht der abgeführte Teil des Fluidgemisches aus Gas oder aus stark mit gasförmigen Bestandteilen angereicherter Flüssigkeit. Der bevorzugt radialsymmetrisch zur Achse des konischen Ringspalts angeordnete Trennabschnitt weist vorzugsweise die Form eines Rohrzylinders auf, kann jedoch zusätzliche, die Abtrennung des ersten Gases begünstigende Elemente aufweisen, wie beispielsweise eine trichterförmige Eingangsöffnung oder einen konisch in Richtung von der Düsenöffnung weg sich aufweitenden Außenmantel.

Die Gaszuführung für das in das flüssige Medium zu dispergierende zweite Gas mündet auf Höhe des Trennabschnitts und radial außenseitig von diesem im Volumen aus. Auf diese Weise wird gewährleistet, dass der Strom des zweiten Gases erst dann dem Fluidgemisch zugegeben wird, wenn die Abtrennung des ersten Gases bereits erfolgt ist. Aufgrund der radial außenseitig am Trennabschnitt des Separators immer sehr noch starken Rotationsbewegung des Fluidstrahls erfolgt eine rasche und intensive Durchmischung des zweiten Gases in das Fluidgemisch, wodurch gegebenenfalls auch ein Einlösen des zweiten Gases begünstigt wird, falls das Gas in der Flüssigkeit lösbar ist.

Der zu behandelnde Strom des Fluidgemisches wird der Düse unter hohem Druck zugeführt, d.h. in der Regel befindet sich die das Fluidgemisch bereits stromauf zur Düse unter einem entsprechenden Druck, oder es wird mittels einer in der Fluidzuführung, stromauf zur Düse angeordneten Pumpe auf einen entsprechenden Druck gebracht. Eine solche Pumpe weist den Vorteil auf, dass sie zu einem definierten Druck am Eingang zum Ringspalt führt; sie kann dabei auch in ihrer Pumpleistung variabel und in Abhängigkeit von gemessenen Parametern regelbar ausgestaltet sein. Hierzu ist die erfindungsgemäße Vorrichtung bevorzugt mit einer beispielsweise elektronischen Steuereinheit ausgestattet, die im Übrigen auch weitere Regelaufgaben im Rahmen der Erfindung übernehmen kann.

Bevorzugt ist als Volumen ein mit dem Fluidgemisch gefüllter, bevorzugt geschlossener Behälter oder eine flüssigkeitsführende Leitung vorgesehen; es kann sich dabei jedoch auch um einen offenen Behälter, ein Becken oder um ein Gewässer handeln. Im Falle einer flüssigkeitsführenden Leitung, etwa einer von dem Fluidgemisch durchströmten Rohrleitung, sind Düse und Trennabschnitt bevorzugt konzentrisch zur Rohrachse angeordnet. Die erfindungsgemäße Vorrichtung kann insbesondere leicht in eine bestehende Rohrleitung eingebaut werden und ermöglicht eine kontinuierliche Behandlung des durch die Rohrleitung geführten Fluidgemischs.

Eine besonders vorteilhafte Ausgestaltung der Erfindung sieht vor, dass in der Ausleitung des Separators ein Phasentrenner, beispielsweise ein Gasphasenseparator, integriert ist, der über eine Rückleitung mit der Fluidzuführung strömungsverbunden ist. Im Phasentrenner wird das erste Gas, das in dem an der Trenneinrichtung aufgenommenen Fluidgemisch enthalten ist, vom übrigen an der Trenneinrichtung aufgenommenen Fluidgemisch getrennt. Die vom ersten Gas befreite flüssige Phase des Fluidgemischs wird anschließend über die Rückleitung zur Fluidzuführung der Düse zurückgeführt. Die Rückführung der flüssigen Phase kann dabei mittels einer in der Rückleitung integrierten Pumpe erfolgen; ebenso ist eine Ausgestaltung der Erfindung möglich, bei der die Rückleitung stromauf einer in der Fluidzuführung angeordneten Pumpe einmündet; in diesem Fall ist in der Rückleitung keine eigene Pumpe erforderlich.

In einer weiterführenden Ausgestaltung der erfindungsgemäßen Vorrichtung sind verschiedene Einstellmöglichkeiten vorgesehen, um die Trennschärfe des Separators den jeweiligen Erfordernissen anpassen zu können. Darunter zählen insbesondere Mittel, um den axialen Abstand zwischen Düsenöffnung und Einlassöffnung des Separators und/oder den axialen Abstand der Einmündung der Gaszuführung für das zweite Gas gegenüber der Düsenöffnung und/oder die axiale Position des Führungskegels der Düse gegenüber der konischen Innenfläche des Düsenmantels zu variieren.

Eine ebenfalls vorteilhafte Ausgestaltung der Erfindung sieht vor, dass in die Fluidzuführung eine Zuleitung einmündet, durch die ein zusätzliches Gas in das Fluidgemisch eingeleitet werden kann. Durch den Partialdruck dieser Komponente kann das zusätzliche Gas als "Sparging-Gas" eingesetzt werden, um die Entgasung des ersten Gases im Fluidstrahl zu unterstützen. Zudem verringert das zusätzlich eingeleitete Gas die Viskosität des Fluidgemisches und erhöht damit die Rotationsgeschwindigkeit des Fluidgemisches an der Düsenöffnung und damit des Fluidstrahls im Volumen. Insbesondere kann auch ein Teilstrom des zweiten Gases auf diese Weise als Sparging-Gas für das erste Gas eingesetzt werden.

In einer ebenfalls vorteilhaften Variante der Erfindung ist die Düse mit einer vor der Düsenöffnung angeordneten Entmischungskammer ausgerüstet. Bei der Entmischungskammer handelt es sich um eine achsensymmetrisch zum konischen Ringspalt vor der Düsenöffnung, d.h. in Fließrichtung des Fluidgemisches gesehen stromab zu dieser, angeordnete zylindrische Kammer mit stirnseitig angeordneten Öffnungen, deren Länge beispielsweise dem 0,5- bis 2-fachen des Führungskegels entspricht und deren führungskegelseitige Eingangsöffnung größer als die dieser gegenüberliegende Ausgangsöffnung ist. Der Separator ist in diesem Fall stromab und beanstandet von der Ausgangsöffnung der Entmischungskammer angeordnet.

Zweckmäßigerweise ist in der Fluidzuführung und/oder im Düsenmantel und/oder im Führungskegel eine Heizeinrichtung vorgesehen. Durch die Beheizung des Fluidgemisches vor und/oder während seines Durchgangs durch die Düse wird seine Viskosität verringert, wodurch insbesondere die Rotationsgeschwindigkeit des Fluidstrahls gesteigert und damit der Druck im Zentrum des Fluidstrahls weiter reduziert werden kann. Generell wird durch die höhere Temperatur die Löslichkeit von im Fluid gelösten Gasen verringert und so ein Ausgasen begünstigt.

Um die Abtrennung des ersten Gases aus dem Fluidgemisch zu unterstützen, ist die Vorrichtung bevorzugt mit einer Ultraschalleinrichtung ausgerüstet. Die Ultraschalleinrichtung kann so aufgebaut sein, dass der Führungskegel und/oder die Düse als Ganzes und/oder der Separator und/oder eine Leitung und/oder ein Behälter, in der bzw. dem die Vorrichtung angeordnet ist, in rasche, ultraschallerzeugende Vibrationen versetzt wird/werden. Während der Vibration bilden sich im Fluidgemisch Zonen mit unterschiedlichem Druck aus, die die Entstehung von Kavitation begünstigen, in denen im Fluidgemisch gelöstes erstes Gas in Form von feinen Gasbläschen ausgast. Die Gasbläschen koagulieren und sammeln sich als Gasphase im Zentrum des rotierenden Fluidstrahls und können anschließend mittels des Separators aus dem Fluidgemisch entfernt werden.

Eine abermals vorteilhafte Ausgestaltung der erfindungsgemäßen Vorrichtung sieht vor, dass durch den Führungskegel zentral eine Ausleitung vorgesehen ist. Über die Ausleitung kann, beispielsweise mittels einer Pumpe, ein Teilstrom des Fluidgemischs aus dem sich vor der Düsenöffnung ausbildenden rotierenden Fluidstrahls abgezogen werden, wodurch der stromab zur Düsenöffnung angeordnete Separator unterstützt wird.

Im Übrigen stehen dem Anwender der erfindungsgemäßen Vorrichtung eine Vielzahl an Parametern zur Verfügung, die er entsprechend der gestellten Aufgabe einstellen kann. Dazu gehören insbesondere die Querschnittsfläche der Düsenöffnung, die Position des Führungskegels in der Düse, der Druck in der Fluidleitung, der Abstand zwischen Düsenöffnung und Separator im Volumen, die Querschnittsfläche einer Eingangsöffnung am Separator, die Form des Separators, die Querschnittsfläche der Düsenöffnung, die Position des Führungskegels in der Düse, der Druck in der Fluidzuführung, der Abstand der Einmündung der Gaszuleitung von der Düsenmündung, die geometrischen Verhältnisse der Gaszuleitung, dem Mengenstrom des an der Gaszuleitung zudosierten zweiten Gases und/oder dem Mengenstrom eines zugeführten Sparging-Gases. Derartige Parameter können ganz oder teilweise von Hand eingestellt oder mittels der bereits erwähnten Steuereinheit automatisch geregelt werden.

Die Aufgabe der Erfindung wird auch durch ein Verfahren mit den Merkmalen des Patentanspruchs 10 gelöst.

Beim erfindungsgemäßen Verfahren erfolgt die Trennung des Fluidgemisches wie folgt: Ein zumindest aus einer Flüssigkeit und mindestens einem darin enthaltenen ersten Gas zusammengesetztes Fluidgemisch wird, beispielsweise unter Einsatz einer Pumpe, über eine Fluidzuleitung einer Düse zugeführt. Das erste Gas, oder die ersten Gase, können dabei ganz oder teilweise in der Flüssigkeit gelöst und/oder in Form kleiner Gasblasen darin dispergiert sein. Die Düse weist einen konischen Ringspalt, also ein von zwei konischen Flächen begrenztes Volumen, auf, wobei das Fluidgemisch über eine tangential in den Ringspalt einmündende Fluidzuführung in den konischen Ringspalt eingespeist wird. Das Fluidgemisch wird im konischen Ringspalt in eine spiralförmig sich verengende Bahn gezwungen und an einer an der Spitze des konischen Ringspalts angeordneten Düsenöffnung in Form eines verdrallten Fluidstrahls in ein mit dem gleichen Fluidgemisch gefülltes Volumen ausgestoßen. Der Druck des Fluidgemisches im Volumen ist geringer als der Druck des zugeführten Fluidgemisches, beispielsweise beträgt er zwischen 0,01 bar und 1,5 bar, bei einem Druck in der Fluidzuführung von beispielsweise zwischen 2 bar und 20 bar, bevorzugt zwischen 2 bar und 5 bar (Angaben jeweils über Umgebungsdruck). Im verdrallten Fluidstrahl bildet sich in radialer Richtung ein Druckgradient aus, der dafür sorgt, dass sich Komponenten geringer Dichte, insbesondere gasförmige Bestandteile, im Zentrum des verdrallten Fluidstrahls anreichern. Die Stärke des Druckgradienten hängt insbesondere von der Druckdifferenz zwischen dem Druck des zugeführten Fluidgemisches und dem Druck im Volumen ab. Sofern das erste Gas gelöst im Fluidgemisch vorliegt, wird der Druck in der Fluidzuleitung bevorzugt so gewählt, dass das erste Gas aus dem Fluidgemisch ausgast und sich im Zentrum des Fluidstrahls konzentriert. Mit Hilfe eines Separators wird ein Teilstrom des Fluidgemisches (hier "Trennstrom" genannt) aus der Zone verringerten Drucks im Zentrum des verdrallten Fluidstrahls abgezogen und vom übrigen Fluidgemisch getrennt. Dabei reicht in der Regel der dynamische Druck des Fluidgemisches im verdrallten Fluidstrahl aus, um den Trennstrom aus dem Volumen abzuführen; optional kann der Separator aber auch mit Mitteln zum Anlegen eines Unterdrucks ausgerüstet sein, etwa einer Pumpe oder einem Leitungssystem, in dem die Gewichtskraft des abgezogenen Fluids zum Erzeugen eines Unterdrucks genutzt wird.

Gleichzeitig mit der zumindest teilweisen Abtrennung des ersten Gases bzw. der ersten Gase aus dem Fluidgemisch wird ein zweites Gas oder werden mehrere zweite Gase in das Fluidgemisch eingetragen. Dies erfolgt erfindungsgemäß durch Eintragen des zweiten Gases oder der zweiten Gase in den verdrallten Strahl des aus der Düse austretenden Fluidgemisches. Um zu verhindern, dass ein Teil des zweiten Gases zusammen mit dem ersten Gas am Separator abgetrennt wird, erfolgt der Eintrag des zweiten Gases bevorzugt erst - in Strömungsrichtung des an der Düse austretenden Fluidstrahls gesehen - auf Höhe der oder stromab zur Eintrittsöffnung des Separators. Dabei vermischt sich das zweite Gas bzw. vermischen sich die zweiten Gase innig mit dem Fluidgemisch. Auch hier ist der Begriff "vermischen" allgemein zu verstehen und kann eine Dispersion von Gasblasen im Fluidgemisch ebenso umfassen wie ein zumindest teilweises Einlösen des Gases.

In einer vorteilhaften Ausgestaltung des erfindungsgemäßen Verfahrens wird dem Fluidgemisch stromauf zur Düsenöffnung ein Sparging-Gas zum Abtrennen des ersten Gases aus dem Fluidgemisch zugeführt. Die Zuführung kann innerhalb der Düse oder stromauf zur Düse erfolgen. Durch das Sparging-Gas wird die Abtrennung des ersten Gases aus dem Fluidgemisch unterstützt. Beispielsweise kann Stickstoff als Sparging-Gas zum Entfernen von Sauerstoff aus dem Fluidgemisch eingesetzt werden.

Als Sparging-Gas kommt bevorzugt ein Inertgas und/oder das zweite Gas zum Einsatz. Im letzteren Fall wird beispielsweise ein aus einer gemeinsamen Quelle entstammender Strom des zweiten Gases in zwei Teilströme zerlegt, von denen einer der Gaszuführung zugeführt, der andere jedoch stromauf zu Düsenöffnung als Sparging-Gas in die Fluidzuführung für das Fluidgemisch eingebracht wird.

Wird das Sparging-Gas einer Quelle entnommen, in der es tiefkalt oder komprimiert vorliegt, kann das Sparging-Gas in vorteilhafter Weise zur Kühlung eingesetzt werden, indem seine Verdampfungs- bzw. Entspannungsenthalpie dem bereits abgetrennten ersten Gas entzogen wird. Auf diese Weise können beispielsweise im ersten Gas enthaltene Stoffe durch kryogene Kondensation abgetrennt werden. Beispielsweise kann in der Weinherstellung so verhindert werden, dass wichtige flüchtige Aromastoffe beim Entgasen des Weines verloren gehen. Die aus dem ersten Gas abgetrennten Stoffe können in einem anschließenden Schritt erneut dem Fluidgemisch, im Beispiel also dem entgasten Wein, zugeführt werden.

Eine bevorzugte Ausgestaltung der Erfindung sieht vor, dass die Düse und der Separator in einem Teilkreislauf für das Fluidgemisch integriert sind. Nach Durchlaufen des Separators wird der Trennstrom einem Phasenseparator zugeführt und seinerseits einer Trennung unterzogen, in der das erste Gas oder die ersten Gase vom übrigen Fluidgemisch abgetrennt werden, sofern nicht bereits im Separator eine vollständige Trennung erfolgt ist. Das verbleibende, weitgehend vom ersten Gas oder den ersten Gasen befreite Fluidgemisch wird anschließend über eine Rückleitung in den Vorlauf der Düse zurückgeführt. In der Rückleitung kann dabei eine Fördereinrichtung, beispielsweise eine elektrische Pumpe, angeordnet sein, mittels der kontinuierlich Fluidgemisch aus dem Separator in die Fluidzuführung stromauf zur Düse eingespeist werden kann.

Die erfindungsgemäße Vorrichtung und das erfindungsgemäße Verfahren ermöglicht einen kontinuierlichen Gasaustausch, bei dem also zeitgleich ein im Fluidgemisch enthaltenes erstes Gas entfernt und ein zweites Gas in das Fluidgemisch eingespeist wird. Sie eignen sich insbesondere zum Behandeln von homogenen oder inhomogenen Fluidgemischen, wie Lösungen oder Suspensionen und ist auch bei hitzeempfindlichen Fluidgemischen einsetzbar. Beispielsweise sind sie für die eingangs erwähnten biologischen, lebensmitteltechnischen oder medizinischen Anwendungen geeignet. Bei der im Fluidgemisch enthaltenen Flüssigkeit handelt es sich beispielsweise um Wasser oder um eine wässerige Lösung oder Suspension aus einem mikrobiologischen oder lebensmitteltechnischen Prozess, oder um Öl. Beispielsweise handelt es sich um Wein oder um ein anderes Getränk. Beispielsweise handelt es sich um Wasser, das hinsichtlich seines Sauerstoffgehalts zu entgasen und gleichzeitig zu karbonisieren ist. Die Erfindung ist freilich nicht auf diese Beispiele beschränkt, sondern in einer Vielzahl weiterer Anwendungen einsetzbar.

Gegenüber den im Stande der Technik bekannten Verfahren und Vorrichtungen kommt die Erfindung mit einem vergleichsweise geringen Energieeinsatz aus, benötigt keine zusätzlichen Substanzen zur Durchführung der Trennung und ist schonend für das zu behandelnde Fluidgemisch sowie das eingesetzte Material. Die erfindungsgemäße Vorrichtung kommt ohne Filter, Membranen, bewegliche Teile oder sonstige Verschleißteile und arbeitet im Einsatz geräuscharm.

Anhand der Zeichnungen sollen Ausführungsbeispiele der Erfindung näher erläutert werden. In schematischen Ansichten zeigen:
- Fig. 1:: Eine erfindungsgemäße Vorrichtung in einer ersten Ausführungsform im Längsschnitt,
- Fig. 2:: Die Vorrichtung aus Fig. 1 im Querschnitt längs der Schnittlinie B-B in Fig. 1,
- Fig. 3: Eine erfindungsgemäße Vorrichtung in einer anderen Ausführungsform im Längsschnitt.

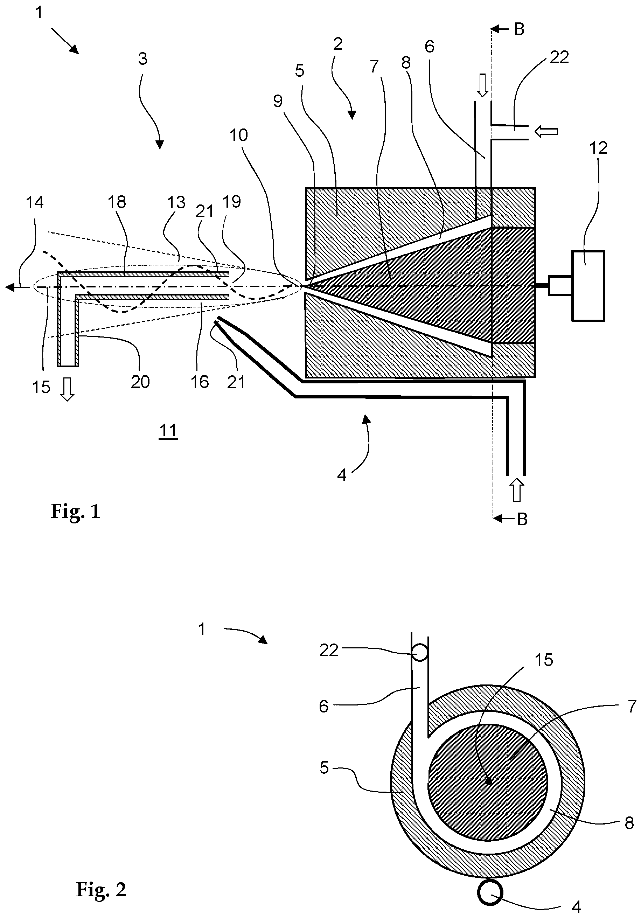

Die in den Fig. 1 und 2 gezeigte Vorrichtung 1 dient zur Behandlung eines eine Flüssigkeit und ein erstes Gas enthaltenden Fluidgemisches. Bei der Behandlung wird kontinuierlich das erste Gas zumindest teilweise aus dem Fluidgemisch entfernt und gleichzeitig ein zweites Gas in das Fluidgemisch dispergiert. Beispielsweise handelt es sich bei der Flüssigkeit im Fluidgemisch um Wasser oder um eine wässerige Lösung, beim ersten Gas um ein in der Flüssigkeit gelöstes Gas und beim zweiten Gas um ein in der Flüssigkeit zu lösendes Gas.

Die Vorrichtung 1 umfasst eine Düse 2 zum Eintragen des Fluidgemisches, einen axial beabstandet vor dieser angeordneten Separator 3 zum Entfernen des ersten Gases und eine Gaszuführung 4 zum Einleiten des zweiten Gases.

Die Düse 2 umfasst einen Düsenmantel 5 mit einer konischen Innenfläche, in den tangential eine Fluidzuführung 6 einmündet. Innerhalb des Düsenmantels 5 ist ein gleichfalls konisch geformter Führungskegel 7 derart angeordnet, dass zwischen der Innenwand des Düsenmantels 5 und der Außenwand des Führungskegels 7 ein konischer Ringspalt 8 offensteht und bevorzugt derart, dass die Kegelspitze 9 des Führungskegels 7 im Wesentlichen mit einer Düsenöffnung 10 der Düse 2 fluchtet. Die Strömungsquerschnitte von Düsenöffnung 10, Ringspalt 8 und Fluidzuführung 6 sind bevorzugt im Wesentlichen gleich groß gewählt. Die Innenfläche des konischen Düsenmantels 5 und die Außenfläche des Führungskegels 7 können dabei einen gleichen Öffnungswinkel aufweisen, es ist jedoch auch vorstellbar, dass der Öffnungswinkel der Außenfläche des Führungskegels 7 spitzer als der Öffnungswinkel der Innenfläche des Düsenmantels 5 ist, sich also der radiale Abstand von Düsenmantel 5 und Führungskegel 7 in Richtung zur Düsenöffnung 10 hin reduziert, wie in Fig. 1 gezeigt.

Düse 2, Separator 3 und Gaszuleitung 4 sind innerhalb eines Volumens 11 aufgenommen, bei dem es sich beispielsweise um einen Behälter oder um eine Leitung handelt und das mit dem zu behandelnden Fluidgemisch gefüllt ist.

Der Führungskegel 7 kann innerhalb des Düsenmantels 5 fest montiert oder - wie hier gezeigt - mittels einer manuellen oder motorgetriebenen Verschiebeeinrichtung 12 axial beweglich aufgenommen sein, um die Vorrichtung 1 an die Eigenschaften des zu behandelnden Fluidgemisches und/oder die jeweilige Arbeitsaufgabe anzupassen.

Im Betrieb der Düse 2 wird ein zu behandelndes Fluidgemisch, beispielsweise eine zu entgasende Flüssigkeit, mit einem Druck von beispielsweise 2-5 bar über die Fluidzuführung 6 in Pfeilrichtung in den Ringspalt 8 eingeleitet. Im Ringspalt 8 wird das Fluidgemisch in eine schnelle Rotationsbewegung versetzt, deren Winkelgeschwindigkeit aufgrund des sich in Fließrichtung verringernden Radius des Ringspalts 8 bis zur Düsenöffnung 10 zunimmt. Aus dem gleichen Grund nimmt auch die in Richtung der Düsenöffnung 10 gerichtete lineare Geschwindigkeitskomponente zu. Das Fluidgemisch verlässt die Düse 2 an der Düsenöffnung 10 und wird im Volumen 11, in dem beispielsweise ein Druck von 1 bar herrscht, als stark verdrallter Fluidstrahl 13 mit großem Drehmoment und hoher axialer Geschwindigkeit in Richtung des Pfeils 14 eingetragen. Aufgrund der hohen Rotationsgeschwindigkeit entsteht längs einer zentralen Achse 15 des Fluidstrahls 13, die zugleich die Symmetrieachse des Ringspalts 7 ist, eine Zone 16 stark verminderten Drucks. In der Zone 16 sammelt sich im Fluidgemisch enthaltenes, beispielsweise ausgasendes gelöstes Gas in Form einer Vielzahl kleiner Gasblasen oder einer einzigen Gasblase an und trennt sich auf diese Weise zumindest teilweise von der im Fluidgemisch enthaltenen Flüssigkeit.

In die Zone 16 taucht der Separator 3 mit einem Trennabschnitt 18 ein. Beim Trennabschnitt 18 handelt es sich in dem in den Fig. 1, 2 gezeigten Ausführungsbeispiel um einen Rohrzylinder, der radialsymmetrisch zur Achse 15 im Volumen 11 angeordnet ist und dessen vordere Einlassöffnung 19 von der Düsenöffnung 8 axial beabstandet ist. Im Ausführungsbeispiel ist die Querschnittsfläche der Einlassöffnung 19 größer als die der der Düsenöffnung 10, sie kann jedoch auch gleich groß oder kleiner gewählt sein. Der Trennabschnitt 18 ist mit einer Ausleitung 20 strömungsverbunden, über die im Trennabschnitt 18 eindringendes Fluidgemisch aus dem Volumen 11, gegebenenfalls mittels einer hier nicht gezeigten Pumpe abgezogen wird; eine Pumpe ist zu diesem Zweck jedoch nicht zwingend erforderlich.

In größerem radialen Abstand von der Achse 15 und radial außerhalb des Trennabschnitts 18 findet sich somit Fluidgemisch, das im Wesentlichen frei von Bestandteilen des ersten Gases ist. In dieses Fluidgemisch wird mittels der Gaszuführung 4 ein zweites Gas eingetragen. Das zweite Gas wird dazu aus einer hier nicht gezeigten Quelle, beispielsweise einem Tank, herangeführt und tritt an einer Ausmündung 21 der Gaszuführung 4 in das im Volumen 11 befindliche Fluidgemisch ein. Um zu verhindern, dass das zweite Gas in die Zone 16 mit geringem Druck gelangt und zusammen mit dem ersten Gas aus dem Fluidgemisch entfernt wird, befindet sich die Ausmündung 21 auf Höhe des Trennabschnitts 18 und radial beabstandet zu diesem. Das an der Ausmündung 21 austretende Gas gelangt so in den äußeren Bereich des verdrallten Fluidstrahls 13 und wird dort intensiv mit dem umgebenden Fluidgemisch vermischt. Um die Effizienz des Gaseintrags noch zu erhöhen, kann die Gaszuführung 4, wie in Fig. 1 gezeigt, im Bereich der Ausmündung 21 düsenförmig, also mit einem sich konisch verjüngendem Querschnitt, ausgebildet sein.

Im Übrigen kann die Vorrichtung 1 im Volumen 11 horizontal oder vertikal angeordnet sein; insbesondere kann durch eine vertikale Anordnung in einem der Ausleitung 20 nachgeschalteten Gasphasentrenner (in Fig. 1 nicht gezeigt) das abgetrennte Gas nach oben hin entweichen, während die im Gasphasentrenner verbleibende flüssige Phase zur Fluidzuführung 5 zurückgeführt werden kann. Sofern das zweite Gas in der Quelle im tiefkalt verflüssigten Zustand oder unter Druck gelagert wurde, kann die zum Verdampfen oder Entspannen des zweiten Gases erforderliche Energie mittels eines hier nicht gezeigten Wärmetauschers anderen Fluidströmen entnommen werden, beispielsweise dem über die Ausleitung 20 abgeführten ersten Gas.

Um die Entfernung des ersten Gases aus dem Fluidgemisch zu unterstützen, kann bei der Vorrichtung 1 über eine in die Fluidzuführung einmündende Gaszuleitung 22 ein Sparging-Gas in das der Düse 2 zugeführte Fluidgemisch eingeleitet werden. Beim Sparging-Gas handelt es sich insbesondere um einen Teilstrom des zweiten Gases, dessen übriger Strom über die Gaszuführung 4 in das Fluidgemisch eingetragen wird. Anstelle des zweiten Gases kann jedoch auch ein anderes Gas, insbesondere ein inertes Gas, beispielsweise Stickstoff oder ein Edelgas, als Sparging-Gas zum Einsatz kommen.

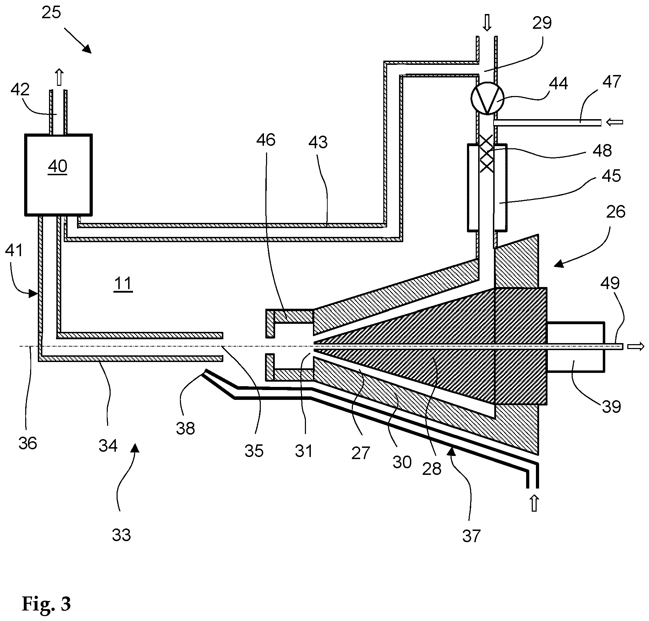

Fig. 3 zeigt eine erfindungsgemäße Vorrichtung 25 in einer anderen Ausführungsform.

In ähnlicher Weise wie die Vorrichtung 1 weist die in Fig. 3 gezeigte Vorrichtung 25 eine Düse 26 mit einem konischen Ringspalt 27 auf, der zwischen einem axialbeweglich angeordneten Führungskegel 28 und einem konischen Düsenmantel 30 angeordnet ist und in den eine Fluidzuführung 29 einmündet. An der Spitze des konischen Ringspalts 27 ist eine Düsenöffnung 31 vorgesehen.

Ebenso wie die Vorrichtung 1 weist die Vorrichtung 25 einen Separator 33 mit einem rohrzylinderförmigen Trennabschnitt 34 auf, der entlang einer Achse 36 axial beabstandet vor der Düse 27 angeordnet ist und dessen der Düse 27 gegenüberliegenden Stirnseite eine Einlassöffnung 35 aufweist, sowie eine Gaszuleitung 37, der seitlich zum Trennabschnitt 34 mit einer Ausmündung 38 ausmündet.

Weiterhin kann die Vorrichtung 25 mit einem Ultraschallerzeuger 39 ausgestattet sein, mittels dessen beispielsweise der Führungskegel 28 in Schwingungen sehr hoher Frequenz (Ultraschall-Schwingungen) versetzt werden kann. Dadurch werden stromab zur Düsenöffnung 31 im zu behandelnden Fluidgemisch Kavitationen erzeugt, in die hinein gelöstes erstes Gas aus dem Fluidgemisch ausgasen kann. Dadurch wird die Effizienz der Vorrichtung 25 weiter gesteigert. Im Übrigen kann anstelle des Führungskegels 28 auch beispielsweise die ganze Düse 25 oder der den Führungskegel 28 umschließende Düsenmantel in Ultraschall-Schwingungen versetzt werden.

Die Vorrichtung 25 verfügt zudem über einen Phasentrenner 40, in dem das im Separator 33 abgetrennte und über eine Ausleitung 41 dem Phasentrenner 40 zugeführte, stark mit dem ersten Gas angereicherte Fluidgemisch in eine Gasphase und eine flüssige Phase getrennt wird. Während die Gasphase über eine Gasableitung 42 abgeführt wird, wird die vom Gas befreite flüssige Phase über eine Rückleitung 43 erneut in die Fluidzuführung 29, stromauf zu einer das unbehandelte Fluidgemisch zur Düse 26 fördernden Pumpe 44, eingespeist. In der Fluidzuführung 29 ist in der Vorrichtung 25 zusätzlich ein Heizelement 45, beispielsweise eine elektrische Heizeinrichtung, angeordnet, mittels der das Fluidgemisch erwärmt und damit seine Viskosität herabgesetzt werden kann. Weiterhin kann stromab zur Düsenöffnung 31 eine rohrförmige Entmischungskammer 46 angeordnet sein, die die Auftrennung des aus der Düse 26 austretenden Fluidgemisches begünstigt. Länge und Querschnitt der Entmischungskammer 46 werden dabei so gewählt, dass ein für das jeweilige Fluidgemisch guter Trennungseffekt erzielt wird.

Um die Trennung des ersten Gases von der Flüssigkeit zu unterstützen, kann auch in der Vorrichtung 25 ein Sparging-Gas, beispielsweise Stickstoff oder ein anderes Inertgas, eingesetzt werden. Dazu weist die Vorrichtung 25 eine in die Fluidzuleitung 29 stromab zur Pumpe 44 einmündende Gaszuleitung 47 sowie ein stromab zur Einmündung der Gaszuleitung 47 in der Fluidzuleitung 29 eingebautes Mischelement 48 auf. Das Mischelement 48 dient zum intensiven Durchmischen des über die Gaszuleitung 47 eingetragenen Sparging-Gases mit dem umgebenden, über die Fluidzuleitung 29 zugeführten Fluidgemisch.

Der Betrieb der Vorrichtung 25 erfolgt ähnlich dem der Vorrichtung 1. Ein Fluidgemisch, beispielsweise Wasser, in welcher ein erstes Gas, beispielsweise Sauerstoff, in gelöster oder eingemischter Form vorliegt, wird über die Fluidzuleitung 29 mittels der Pumpe 44 zur Düse transportiert und dabei mit einem über die Gaszuleitung 45 zugeführten Sparging-Gas angereichert. Das Fluidgemisch wird im Ringspalt 27 der Düse 26 in eine rasche Rotationsbewegung versetzt und verlässt die Düsenöffnung 31 in Gestalt eines stark verdrallten Fluidstrahls, wobei sie das im Volumen 11 bereits vorliegende Fluidgemisch verdrängt. Im verdrallten Fluidstrahl sorgt ein starker Druckgradient in radialer Richtung dafür, dass sich im Fluidgemisch gelöste oder eingemischte Bestandteile des ersten Gases sowie des Sparging-Gases in einer gasreichen Phase im Zentrum des Stahls entlang der Achse 36 anreichern. Der geringe Partialdruck des ersten Gases in Gasblasen des Sparging-Gases fördert dabei das Ausgasen des ersten Gases aus der umgebenden Flüssigkeit des Fluidgemischs. Die zentrale gasreiche Phase wird im Separator 33 von der umgebenden, vom ersten Gas weitgehend befreiten Phase des Fluidgemischs getrennt. Im Phasentrenner 40 wird die separierte gasreiche Phase weiter in eine Gasphase und eine flüssige Phase aufgetrennt. Während die Gasphase über die Gasableitung 42 abgeführt wird, wird die abgetrennte Flüssigkeit über die Rückleitung 43 erneut in die Fluidzuleitung 29 eingespeist. Gleichzeitig mit der Entfernung des ersten Gases wird über die Gaszuleitung 37 ein zweites Gas, beispielsweise Kohlendioxid, in einen Bereich im Volumen 11 eingetragen, in dem das Fluidgemisch bereits weitgehend vom ersten Gas befreit ist, beispielsweise in einen Bereich radial außenseitig am Trennabschnitt 34. Durch die dort weiterhin starke Drallströmung des Fluidgemischs wird das zweite Gas innig mit dem Fluidgemisch durchmischt.

Alternativ oder ergänzend zu einem vor der Mündungsöffnung 31 der Düse 26 im Volumen 11 angeordneten Separator 34 kann im Übrigen die gasreiche Phase im Zentrum des sich vor der Mündungsöffnung 31 der Düse 26 auch über eine zentral durch den Führungskegel 28 geführten Ausleitung 49, beispielsweise mittels einer hier nicht gezeigten Pumpe, abgezogen und auf diese Weise vom übrigen Fluidgemisch getrennt werden.

Die Erfindung ist im Übrigen nicht auf die hier gezeigten Ausführungsbeispiele beschränkt. Vielmehr können verschiedene Merkmale der Vorrichtungen 1, 25 beliebig miteinander kombiniert oder die Vorrichtungen 1, 25 um weitere Merkmale ergänzt werden. Beispielsweise kann eine Heizeinrichtung oder ein Ultraschallerzeuger auch in der Vorrichtung 1 vorgesehen sein. Ebenso können verschiedene Betriebsparameter einer erfindungsgemäßen Vorrichtung, wie beispielsweise die Heizleistung des Heizelements 30, die Breite des Ringspalts 7, 27, der axiale Abstand des Separators 3, 33 oder der Ausmündung 21, 38 von der Düse 2, 26 oder der Druck einer das zu behandelnde Fluidgemisch zur Düse 2, 26 fördernden Pumpe mittels einer hier nicht gezeigten elektronischen Steuerung in Abhängigkeit von kontinuierlich gemessenen Parametern, wie beispielsweise des Konzentrationen des ersten und/oder zweiten Gases in dem behandelten Fluidgemisch, stromab zu Separator 3, 33 und Gaszuführung 21, 37, geregelt werden.

### Bezugszeichenliste

| | | | |
|---|---|---|---|
| 1 | Vorrichtung | 26 | Düse |
| 2 | Düse | 27 | Ringspalt |
| 3 | Separator | 28 | Führungskegel |
| 4 | Gaszuleitung | 29 | Fluidzuführung |
| 5 | Düsenmantel | 30 | Düsenmantel |
| 6 | Fluidzuführung | 31 | Düsenöffnung |
| 7 | Führungskegel | 32 | - |
| 8 | Ringspalt | 33 | Separator |
| 9 | Kegelspitze | 34 | Trennabschnitt |
| 10 | Düsenöffnung | 35 | Einlassöffnung |
| 11 | Volumen | 36 | Achse |
| 12 | Verschiebeeinrichtung | 37 | Gaszuleitung |
| 13 | Fluidstrahl | 38 | Ausmündung |
| 14 | Pfeil | 39 | Ultraschallerzeuger |
| 15 | Achse | 40 | Phasentrenner |
| 16 | Zone | 41 | Ausleitung |
| 17 | - | 42 | Gasableitung |
| 18 | Trennabschnitt | 43 | Rückleitung |
| 19 | Einlassöffnung | 44 | Pumpe |
| 20 | Ausleitung | 45 | Heizelement |
| 21 | Ausmündung | 46 | Entmischungskammer |
| 22 | Gaszuleitung | 47 | Gaszuleitung |
| 23 | - | 48 | Mischelement |
| 24 | - | 49 | Ausleitung |
| 25 | Vorrichtung | | |

## Patentansprüche

1. Vorrichtung zum kontinuierlichen Gasaustausch in einem Strom eines Fluidgemischs, mit einer Düse (2, 26) zum Zuführen eines aus mehreren Komponenten zusammengesetzten Fluidgemisches in ein im Einsatz der Vorrichtung vom Fluidgemisch gefülltes Volumen (11), welche Düse (2, 26) einen zwischen einer konischen Innenfläche eines Düsenmantels (5, 30) und einem Führungskegel (7, 28) angeordneten, an seiner Spitze an einer Düsenöffnung (10, 31) in das Volumen (11) ausmündenden konischen Ringspalt (8, 27) und eine tangential in den konischen Ringspalt (8, 27) einmündende Fluidzuführung (6, 29) aufweist, sowie mit einem Separator (3, 33) zum Abtrennen eines im Fluidgemisch enthaltenen ersten Gases, der einen im Volumen (11) angeordneten, mit einer Ausleitung (20, 41) strömungsverbundenen Trennabschnitt (18, 34) mit einer konzentrisch zur Düsenöffnung (10, 31) und axial beabstandet von dieser angeordneten Einlassöffnung (19) aufweist, sowie mit wenigstens einer, mit einer Gasquelle strömungsverbundenen Gaszuführung (3, 37) für ein in das Fluidgemisch zu dispergierendes zweites Gas, die beabstandet von der Düsenöffnung (10, 31) auf Höhe des Trennabschnitts (18, 34) und radial außenseitig von diesem an einer Ausmündung (21, 38) in das Volumen (11) einmündet.

2. Vorrichtung nach Anspruch 1, **dadurch gekennzeichnet, dass** als Volumen (11) ein mit dem Fluidgemisch gefüllter Behälter oder eine das Fluidgemisch führende Leitung vorgesehen ist.

3. Vorrichtung nach Anspruch 1 oder 2, **dadurch gekennzeichnet, dass** in der Ausleitung (20, 41) des Separators (3, 33) ein Phasentrenner (40) integriert ist, der über eine Rückleitung (43) mit der Fluidzuführung (6, 29) strömungsverbunden ist.

4. Vorrichtung nach einem der vorhergehenden Ansprüche, **dadurch gekennzeichnet, dass** der axiale Abstand zwischen Düsenöffnung (10, 31) und Einlassöffnung (19, 19a, 19b, 19c, 19d) des Separators (3, 33) und/oder der axiale Abstand zwischen Düsenöffnung (10, 31) und Ausmündung (21, 38) der Gaszuführung (4, 37) für das zweite Gas und/oder die axiale Position des Führungskegels (7, 28) der Düse (2, 26) gegenüber der konischen Innenfläche des Düsenmantels (5, 30) verstellbar ist.

5. Vorrichtung nach einem der vorhergehenden Ansprüche, **dadurch gekennzeichnet, dass** in die Fluidzuführung (6, 29) und/oder in den Ringspalt (8, 27) eine Gaszuleitung (22, 47) für ein Sparging-Gas einmündet.

6. Vorrichtung nach einem der vorhergehenden Ansprüche, **dadurch gekennzeichnet, dass** die Düse (2, 26), stromab zur Düsenöffnung (10, 31), mit einer Entmischungskammer (46) ausgerüstet ist.

7. Vorrichtung nach einem der vorhergehenden Ansprüche, **dadurch gekennzeichnet, dass** in der Fluidzuführung (6, 29), im Düsenmantel (5, 30) und/oder im Führungskegel (7, 28) eine Heizeinrichtung (30) vorgesehen ist.

8. Vorrichtung nach einem der vorhergehenden Ansprüche, **gekennzeichnet durch** einen Ultraschallerzeuger (39) zur Erzeugung kurzzeitiger, lokaler Druckunterschiede im Fluidgemisch.

9. Vorrichtung nach einem der vorhergehenden Ansprüche, **dadurch gekennzeichnet, dass** durch den Führungskegel (7, 28) zentral eine Ausleitung (49) zum Abführen eines Teilstroms des stromab zur Düsenöffnung (10, 31) vorliegenden Fluidgemisches vorgesehen ist.

10. Verfahren zum kontinuierlichen Gasaustausch in einem Strom eines Fluidgemischs, bei dem
a. ein wenigstens eine Flüssigkeit und wenigstens ein erstes Gas enthaltendes Fluidgemisch einer Düse (2, 26) zugeführt wird, die einen konischen Ringspalt (8, 27) aufweist, wobei das Fluidgemisch über eine tangential in den Ringspalt (8, 27) einmündende Fluidzuführung (6, 29) in den konischen Ringspalt (8, 27) eingespeist wird,
b. das Fluidgemisch im konischen Ringspalt (8, 27) in eine spiralförmig sich verengende Bahn gezwungen und an einer an der Spitze des konischen Ringspalts (8, 27) angeordneten Düsenöffnung (10, 31) in Form eines verdrallten Fluidstrahls (13) in ein Volumen (11) ausgestoßen wird, wobei sich im verdrallten Fluidstrahl eine Zone (16) verringerten Drucks ausbildet und sich das im Fluidgemisch enthaltene erste Gas in der Zone (16) verringerten Drucks anreichert,
c. mit Hilfe eines im Volumen, vor der Düsenöffnung (10, 33), angeordneten Separators (3, 33) ein mit dem ersten Gas angereicherter Teilstrom (Trennstrom) des Fluidgemisches aus der Zone (16) verringerten Drucks abgezogen und von einem Reststrom des Fluidgemisches getrennt wird, und
d. in den Reststrom mittels einer Gaszuführung (4) ein Strom eines zweiten Gases dispergiert wird.

11. Verfahren nach Anspruch 10, **dadurch gekennzeichnet, dass** dem Fluidgemisch stromauf zur Düsenöffnung (10, 31) ein Sparging-Gas zum Abtrennen des ersten Gases aus dem Fluidgemisch zugeführt wird.

12. Verfahren nach Anspruch 11, **dadurch gekennzeichnet, dass** als Sparging-Gas ein Strom des zweiten Gases zum Einsatz kommt.

13. Verfahren nach einem der Ansprüche 11 oder 12, **dadurch gekennzeichnet, dass** als Sparging-Gas ein tiefkalt verflüssigtes oder unter Druck stehendes Gas zum Einsatz kommt, dessen Kälteinhalt zum Kühlen des Trennstroms und/oder einer im Separator (3, 33) separierten Gasphase eingesetzt wird.

14. Verfahren nach einem der Ansprüche 10 bis 13, **dadurch gekennzeichnet, dass** der aus der Zone (16) abgezogene Trennstrom des Fluidgemisches zum Abtrennen des ersten Gases einer Phasenseparation zum Abtrennen des ersten Gases von einer flüssigen Phase unterworden und die flüssige Phase erneut der Düse (2, 26) zugeführt wird.

## Claims

1. Device for continuous gas exchange in a stream of a fluid mixture, with a nozzle (2, 26) for supplying a fluid mixture composed of several components into a volume (11) filled by the fluid mixture when the device is in use, which nozzle (2, 26) has a conical annular gap (8, 27) arranged between a conical inner surface of a nozzle jacket (5, 30) and a guide cone (7, 28) and opening at its tip, at a nozzle opening (10, 31), into the volume (11), and a fluid supply line (6, 29) opening tangentially into the conical annular gap (8, 27), and with a separator (3, 33) for separating a first gas contained in the fluid mixture, which separator has a separating portion (18, 34) arranged in the volume (11) and fluidically connected to an outlet line (20, 41), with an inlet opening (19) arranged concentrically with respect to the nozzle opening (10, 31) and axially spaced apart from the latter, and with at least one gas supply line (3, 37) which is fluidically connected to a gas source for a second gas to be dispersed into the fluid mixture and which, at an outlet (21, 38), opens into the volume (11) at a distance from the nozzle opening (10, 31), at the level of the separating portion (18, 34) and radially to the outside of the latter.

2. Device according to Claim 1, **characterized in that** a container filled with the fluid mixture or a line carrying the fluid mixture is provided as the volume (11).

3. Device according to Claim 1 or 2, **characterized in that** a phase separator (40) is integrated in the outlet line (20, 41) of the separator (3, 33) and is fluidically connected to the fluid supply line (6, 29) via a return line (43).

4. Device according to any one of the preceding claims, **characterized in that** the axial distance between the nozzle opening (10, 31) and the inlet opening (19, 19a, 19b, 19c, 19d) of the separator (3, 33) is adjustable, and/or the axial distance between the nozzle opening (10, 31) and the outlet (21, 38) of the gas supply line (4, 37) for the second gas is adjustable, and/or the axial position of the guide cone (7, 28) of the nozzle (2, 26) relative to the conical inner surface of the nozzle jacket (5, 30) is adjustable.

5. Device according to any one of the preceding claims, **characterized in that** a gas supply line (22, 47) for a sparging gas opens into the fluid supply line (6, 29) and/or into the annular gap (8, 27).

6. Device according to any one of the preceding claims, **characterized in that** the nozzle (2, 26) is equipped, downstream of the nozzle opening (10, 31), with a separation chamber (46).

7. Device according to any one of the preceding claims, **characterized in that** a heating device (30) is provided in the fluid supply line (6, 29), in the nozzle jacket (5, 30) and/or in the guide cone (7, 28).

8. Device according to any one of the preceding claims, **characterized by** an ultrasound generator (39) for generating short-term, local pressure differences in the fluid mixture.

9. Device according to any one of the preceding claims, **characterized in that** an outlet line (49) is provided centrally through the guide cone (7, 28) for discharging a partial stream of the fluid mixture present downstream of the nozzle opening (10, 31).

10. Method for continuous gas exchange in a stream of a fluid mixture, in which method
a. a fluid mixture containing at least one liquid and at least one first gas is supplied to a nozzle (2, 26) which has a conical annular gap (8, 27), wherein the fluid mixture is fed into the conical annular gap (8, 27) via a fluid supply line (6, 29) which opens tangentially into the annular gap (8, 27),
b. the fluid mixture in the conical annular gap (8, 27) is forced into a helically narrowing path and, at a nozzle opening (10, 31) arranged at the tip of the conical annular gap (8, 27), is ejected in the form of a twisted fluid jet (13) into a volume (11), wherein a zone (16) of reduced pressure forms in the twisted fluid jet, and the first gas contained in the fluid mixture enriches in the zone (16) of reduced pressure,
c. with the aid of a separator (3, 33) arranged in the volume, upstream of the nozzle opening (10, 33), a partial stream (separation stream) of the fluid mixture enriched with the first gas is withdrawn from the zone (16) of reduced pressure and separated from a residual stream of the fluid mixture, and
d. a stream of a second gas is dispersed into the residual stream by means of a gas supply line (4).

11. Method according to Claim 10, **characterized in that** a sparging gas for separating the first gas from the fluid mixture is supplied to the fluid mixture upstream of the nozzle opening (10, 31).

12. Method according to Claim 11, **characterized in that** a stream of the second gas is used as sparging gas.

13. Method according to either of Claims 11 and 12, **characterized in that** a cryogenically liquefied or pressurized gas is used as sparging gas, the cold content of which is used to cool the separation stream and/or a gas phase separated in the separator (3, 33).

14. Method according to any one of Claims 10 to 13, **characterized in that** the separation stream of the fluid mixture withdrawn from the zone (16) for separating the first gas is subjected to a phase separation for separating the first gas from a liquid phase, and the liquid phase is again supplied to the nozzle (2, 26).

## Revendications

1. Dispositif pour l'échange de gaz continu dans un courant d'un mélange de fluides, avec une buse (2, 26) pour l'amenée d'un mélange de fluides composé de plusieurs composants dans un volume (11) rempli par le mélange de fluides lors de l'utilisation du dispositif, laquelle buse (2, 26) présente une fente annulaire conique (8, 27) agencée entre une surface intérieure conique d'une enveloppe de buse (5, 30) et un cône de guidage (7, 28), débouchant à sa pointe dans le volume (11) au niveau d'une ouverture de buse (10, 31), et une amenée de fluide (6, 29) débouchant tangentiellement dans la fente annulaire conique (8, 27), ainsi qu'avec un séparateur (3, 33) pour la séparation d'un premier gaz contenu dans le mélange de fluides, qui présente une section de séparation (18, 34) agencée dans le volume (11), en liaison d'écoulement avec une sortie (20, 41), avec une ouverture d'entrée (19) agencée concentriquement à l'ouverture de buse (10, 31) et à une distance axiale de celle-ci, ainsi qu'avec au moins une amenée de gaz (3, 37) en liaison d'écoulement avec une source de gaz pour un deuxième gaz à disperser dans le mélange de fluides, qui débouche dans le volume (11) à distance de l'ouverture de buse (10, 31) à hauteur de la section de séparation (18, 34) et radialement à l'extérieur de celle-ci à une embouchure (21, 38).

2. Dispositif selon la revendication 1, **caractérisé en ce qu'**il est prévu en tant que volume (11) un récipient rempli du mélange de fluides ou une conduite conduisant le mélange de fluides.

3. Dispositif selon la revendication 1 ou 2, **caractérisé en ce qu'**un séparateur de phase (40) est intégré dans la sortie (20, 41) du séparateur (3, 33), lequel est en liaison d'écoulement avec l'amenée de fluide (6, 29) via une conduite de retour (43).

4. Dispositif selon l'une quelconque des revendications précédentes, **caractérisé en ce que** la distance axiale entre l'ouverture de buse (10, 31) et l'ouverture d'entrée (19, 19a, 19b, 19c, 19d) du séparateur (3, 33) et/ou la distance axiale entre l'ouverture de buse (10, 31) et l'embouchure (21, 38) de l'amenée de gaz (4, 37) pour le deuxième gaz et/ou la position axiale du cône de guidage (7, 28) de la buse (2, 26) par rapport à la surface intérieure conique de l'enveloppe de buse (5, 30) sont réglables.

5. Dispositif selon l'une quelconque des revendications précédentes, **caractérisé en ce qu'**une conduite d'alimentation en gaz (22, 47) pour un gaz de barbotage débouche dans l'amenée de fluide (6, 29) et/ou dans l'espace annulaire (8, 27).

6. Dispositif selon l'une quelconque des revendications précédentes, **caractérisé en ce que** la buse (2, 26) est équipée, en aval de l'ouverture de buse (10, 31), d'une chambre de démixtion (46).

7. Dispositif selon l'une quelconque des revendications précédentes, **caractérisé en ce qu'**un appareil de chauffage (30) est prévu dans l'amenée de fluide (6, 29), dans l'enveloppe de buse (5, 30) et/ou dans le cône de guidage (7, 28).

8. Dispositif selon l'une quelconque des revendications précédentes, **caractérisé par** un générateur d'ultrasons (39) pour générer des différences de pression locales de courte durée dans le mélange de fluides.

9. Dispositif selon l'une quelconque des revendications précédentes, **caractérisé en ce qu'**une sortie (49) est prévue centralement à travers le cône de guidage (7, 28) pour évacuer un courant partiel du mélange de fluides présent en aval de l'ouverture de buse (10, 31).

10. Procédé d'échange de gaz continu dans un courant d'un mélange de fluides, dans lequel
a. un mélange de fluides contenant au moins un liquide et au moins un premier gaz est amené à une buse (2, 26) qui présente une fente annulaire conique (8, 27), le mélange de fluides étant introduit dans la fente annulaire conique (8, 27) via une amenée de fluide (6, 29) débouchant tangentiellement dans la fente annulaire (8, 27),
b. le mélange de fluides est forcé dans la fente annulaire conique (8, 27) dans une trajectoire se rétrécissant en spirale et est éjecté dans un volume (11) sous la forme d'un jet de fluide torsadé (13) au niveau d'une ouverture de buse (10, 31) agencée à la pointe de la fente annulaire conique (8, 27), une zone (16) de pression réduite se formant dans le jet de fluide torsadé et le premier gaz contenu dans le mélange de fluides se concentrant dans la zone (16) de pression réduite,
c. à l'aide d'un séparateur (3, 33) agencé dans le volume, avant l'ouverture de buse (10, 33), un courant partiel (courant de séparation) du mélange de fluides enrichi avec le premier gaz est soutiré de la zone (16) de pression réduite et séparé d'un courant résiduel du mélange de fluides, et
d. un courant d'un deuxième gaz est dispersé dans le courant résiduel au moyen d'une amenée de gaz (4).

11. Procédé selon la revendication 10, **caractérisé en ce qu'**un gaz de barbotage est amené au mélange de fluides en amont de l'ouverture de buse (10, 31) pour séparer le premier gaz du mélange de fluides.

12. Procédé selon la revendication 11, **caractérisé en ce que** l'on utilise en tant que gaz de barbotage un courant du deuxième gaz.

13. Procédé selon l'une quelconque des revendications 11 ou 12, **caractérisé en ce que** l'on utilise en tant que gaz de barbotage un gaz liquéfié à basse température ou sous pression, dont la teneur en froid est utilisée pour refroidir le courant de séparation et/ou une phase gazeuse séparée dans le séparateur (3, 33).

14. Procédé selon l'une quelconque des revendications 10 à 13, **caractérisé en ce que** le courant de séparation du mélange de fluides soutiré de la zone (16) pour séparer le premier gaz est soumis à une séparation de phase pour séparer le premier gaz d'une phase liquide et la phase liquide est à nouveau amenée à la buse (2, 26).
